(19)

Europäisches
Patentamt

European
Patent Office

Office européen
des brevets

(11) **EP 4 379 337 A1**

(12) **EUROPEAN PATENT APPLICATION**
published in accordance with Art. 153(4) EPC

(43) Date of publication:
**05.06.2024 Bulletin 2024/23**

(21) Application number: **21953838.6**

(22) Date of filing: **20.08.2021**

(51) International Patent Classification (IPC):
*G01L 1/24* (2006.01)

(52) Cooperative Patent Classification (CPC):
**G01L 1/24**

(86) International application number:
**PCT/CN2021/113908**

(87) International publication number:
**WO 2023/019597 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Huawei Technologies Co., Ltd.
Shenzhen, Guangdong 518129 (CN)**

(72) Inventors:
• **WANG, Stephen
Shenzhen, Guangdong 518129 (CN)**

• **LIU, Hongbin
Shenzhen, Guangdong 518063 (CN)**
• **HU, Jian
Tianjin 301900 (CN)**
• **CAO, Danqian
London E3 3TD (GB)**
• **NI, Gang
Shenzhen, Guangdong 518129 (CN)**
• **HUANG, Tao
Shenzhen, Guangdong 518129 (CN)**

(74) Representative: **Pfenning, Meinig & Partner mbB
Patent- und Rechtsanwälte
Theresienhöhe 11a
80339 München (DE)**

(54) **TACTILE SENSING DEVICE, AND DETECTION METHOD AND APPARATUS**

(57)    Disclosed is a tactile sensing device, including a light source, a plurality of optical waveguides, photoelectric sensing components, and a contact point. The light source is disposed at an input end of each optical waveguide, and the photoelectric sensing component is disposed at an output end of each optical waveguide. The plurality of optical waveguides surround the contact point, and the contact point is in contact with each optical waveguide. When there is a contact force at the contact point, the plurality of optical waveguides are deformed, and deformations of the optical waveguides cause losses of optical signals transmitted in the optical waveguides. The photoelectric sensing component obtains an optical signal transmitted by each optical waveguide, and may detect a value and a direction of the contact force based on a variation of the optical signal transmitted by each optical waveguide. A single contact point is used to implement decoupling detection on a force acting on the contact point in different directions to provide a reliable data basis for completing precise manipulation.

FIG. 3

## Description

### TECHNICAL FIELD

This application relates to the field of sensor design, and in particular, to a tactile sensing device and a detection method and apparatus.

### BACKGROUND

With the development of science and technology, intelligent machinery has gradually replaced labor to perform precise manipulation in many fields. Precise tactile information plays a vital role in performing an overall function of the intelligent machinery. A tactile sensor can restore tactile information to a large extent, improve decision-making efficiency of an entire system, and ensure an orderly and safe interaction process.

Currently, the tactile sensor may generally include a capacitive tactile sensor, an inductive tactile sensor, a piezoresistive tactile sensor, a piezoelectric tactile sensor, and the like. However, a single contact point of each of these tactile sensors can typically be used to detect only a force acting on the contact point in a normal direction. How to use a single contact point to implement decoupling detection on a force acting on the contact point in different directions to provide a reliable data basis for completing precise manipulation needs to be resolved urgently.

### SUMMARY

Embodiments of this application provide a tactile sensing device and a detection method and apparatus. A single contact point may be used to implement decoupling detection on a force acting on the contact point in different directions to provide a reliable data basis for completing precise manipulation.

According to a first aspect, an embodiment of this application provides a tactile sensing device, including a light source, a plurality of optical waveguides, photoelectric sensing components, and a contact point. The optical waveguide may include a waveguide layer and a cladding layer. The cladding layer wraps the waveguide layer, and a refractive index of the waveguide layer is greater than a refractive index of the cladding layer. This is to ensure that transmission of an optical signal emitted by the light source in the optical waveguide meets a condition of total reflection. The light source is disposed at an input end of each optical waveguide, and the photoelectric sensing component is disposed at an output end of each optical waveguide. The light source may be a light-emitting diode or infrared light. This is not limited in this embodiment of this application. It should be noted that the plurality of optical waveguides described herein may be some or all of optical waveguides of the tactile sensing device. This is not limited in this embodiment of this application. The plurality of optical waveguides surround the contact point, and the contact point is in contact with each optical waveguide. The contact point may be in contact with most areas of each optical waveguide, or a small part of areas of each optical waveguide. When there is a contact force at the contact point, the plurality of optical waveguides are deformed. Specifically, when there is the contact force at the contact point, each of the plurality of optical waveguides is deformed. The photoelectric sensing component obtains an optical signal transmitted by each optical waveguide, and a variation of the optical signal transmitted by each optical waveguide is used to detect the contact force. In this embodiment of this application, it is found that when forces of different directions and different values are acted on the contact point, the plurality of optical waveguides that surround the contact point and that are in contact with the contact point generate different deformations. In addition, when the optical waveguide is deformed, the total reflection condition of the optical waveguide is destroyed, and the optical signal transmitted in the optical waveguide generates a loss. Therefore, after a mapping relationship between a variation of a luminous flux of each of the plurality of optical waveguides and the contact force at the contact point is obtained in advance, based on the variation of the luminous flux of each of the plurality of optical waveguides and the mapping relationship, the tactile sensing device provided in the first aspect can implement decoupling detection on a force acting on a single contact point in a normal direction and a tangential direction to provide a reliable data basis for completing precise manipulation.

In a possible implementation of the first aspect, the contact point is polygonal, and each side of the contact point is in contact with one optical waveguide. In this implementation, a contact point of a specific shape is provided, so that each side of the contact point can be fully in contact with each optical waveguide, the optical waveguides around the contact point fully surround the contact point, and the optical waveguides around the contact point can fully feel a force acting on the contact point, thereby improving sensitivity of the tactile sensor.

In a possible implementation of the first aspect, the contact point is quadrilateral or triangular. In this implementation, a contact point of a specific shape is provided, so that each side of the contact point can be fully in contact with each optical waveguide, the optical waveguides around the contact point fully surround the contact point, and the optical waveguides around the contact point can fully feel a force acting on the contact point, thereby improving sensitivity of the tactile sensor.

In a possible implementation of the first aspect, a profile of the contact point is arc-shaped. In this implementation, a contact point of a specific shape is provided, which increases structure diversity.

In a possible implementation of the first aspect, a profile of the contact point is circular or elliptical. In this implementation, a contact point of a specific shape is provided, which increases structure diversity.

In a possible implementation of the first aspect, a region between an input end of the optical waveguide and a contact location is of an arc-shaped structure, and the contact location is a location in which the optical waveguide is in contact with the contact point. In this implementation, the optical waveguide is designed to be a gentle light guide structure from the input end to the contact location, to ensure that the optical signal emitted by the light source can have a sufficient strength when reaching the contact location, thereby improving the sensitivity of the tactile sensor.

In a possible implementation of the first aspect, a curvature of an arc is less than a first preset threshold.

In a possible implementation of the first aspect, a region between an input end of the optical waveguide and a contact location is of a linear structure, and the contact location is a location in which the optical waveguide is in contact with the contact point. In this implementation, the optical waveguide is designed to be another gentle light guide structure from the input end to the contact location, to ensure that the optical signal emitted by the light source can have a sufficient strength when reaching the contact location.

In a possible implementation of the first aspect, any at least two optical waveguides in the plurality of optical waveguides intersect.

In a possible implementation of the first aspect, a deviation between a degree of an included angle between two optical waveguides that intersect and 90 degrees is less than a second preset threshold. Interference between optical signals transmitted by different optical waveguides is reduced as much as possible.

In a possible implementation of the first aspect, the plurality of optical waveguides are deployed on a same reference plane. In this implementation, the plurality of optical waveguides are deployed on the same reference plane, so that a size of the tactile sensor can be further reduced. Specifically, a thickness of the tactile sensor can be reduced. It should be noted that, in this implementation, all optical waveguides may be deployed on a same reference plane, or some optical waveguides may be deployed on a same reference plane.

In a possible implementation of the first aspect, the contact point and the plurality of optical waveguides are deployed on the same reference plane. In this implementation, the plurality of optical waveguides and the contact point are deployed on the same reference plane, so that the size of the tactile sensor can be further reduced. Specifically, the thickness of the tactile sensor can be reduced. It should be noted that, in this implementation, all the optical waveguides and the contact point may be deployed on the same reference plane, or some optical waveguides and the contact point may be deployed on the same reference plane.

In a possible implementation of the first aspect, the reference plane is a horizontal plane or an arc-shaped plane.

In a possible implementation of the first aspect, the plurality of optical waveguides are of multiple-input multiple-output structures, and each optical waveguide may exclusively use a light source, or some optical waveguides share one light source.

In a possible implementation of the first aspect, the plurality of optical waveguides are of single-input multi-output (single-input multi-output, SIMO) structures. For example, one light source is disposed at input ends of all the optical waveguides. In this implementation, all the optical waveguides may be of the single-input multiple-output SIMO structures. In this single-input multi-output structure, only one light source needs to be set, thereby reducing components. In addition, a structure is more compact and space is saved.

In a possible implementation of the first aspect, the contact point is of a flexible structure.

In a possible implementation of the first aspect, the tactile sensing device further includes a housing. The light source, the plurality of optical waveguides, and the photoelectric sensing components are accommodated in the housing. A target location of the housing is hollowed out, and the target location corresponds to a location of the contact point.

In a possible implementation of the first aspect, when there is no contact force at the contact point, a deviation between macrobending losses of any two optical waveguides falls within a preset range.

In a possible implementation of the first aspect, a quantity of contact points is not less than 2. In this implementation, the tactile sensor may include a plurality of contact points.

According to a second aspect, an embodiment of this application provides a detection method. The detection method is applied to the tactile sensor described in any one of the first aspect or the possible implementations of the first aspect. The method includes: obtaining, by each photoelectric sensing component, a strength of an optical signal transmitted by each optical waveguide; and obtaining a value and a direction of a contact force based on a first mapping relationship and a variation of the strength of the optical signal transmitted by each optical waveguide, where the first mapping relationship includes mapping between a value of a force of the contact force along a direction of each coordinate axis and the variation of the strength of the optical signal transmitted by each optical waveguide.

In a possible implementation of the second aspect, the obtaining a value and a direction of a contact force based on a first mapping relationship and a variation of the strength of the optical signal transmitted by each optical waveguide includes: when a deviation between a first variation and a second variation falls within a preset range, determining that the direction of the contact force is downward and perpendicular to a plane on which the contact point is located, where the first variation and the second variation are variations of strengths of optical signals transmitted by any two optical waveguides; and obtaining the value of the contact force based on an absolute value of the first variation, an absolute value of the second variation, and the first mapping relationship. If a difference between the optical waveguides is small, it may be considered that when there is a contact force at the contact point, under the action of the contact force, degrees of squeezing of the contact point on the optical waveguides are similar, and consequently, degrees of changes of luminous fluxes of the optical waveguides are similar. This usually occurs when the contact force acting on the contact point has a force only in a normal direction, and does not have a force in a tangential direction. Therefore, if the difference between the optical waveguides is small, it may be considered that the contact force is the force in the normal direction. In addition, in this case, a force along a z-axis direction may be obtained based on a variation of the luminous flux of each optical waveguide and the pre-obtained mapping relationship, and forces along x-axis and y-axis directions may not be obtained, thereby reducing a calculation amount.

In a possible implementation of the second aspect, the obtaining a value and a direction of a contact force based on a first mapping relationship and a variation of the strength of the optical signal transmitted by each optical waveguide includes: when a deviation between a first variation and a second variation does not fall within a preset range, obtaining the value of the force of the contact force along the direction of each coordinate axis based on the first mapping relationship, the first variation, and the second variation, where the first variation and the second variation are variations of strengths of optical signals transmitted by any two optical waveguides; and obtaining the value and the direction of the contact force based on the value of the force of the contact force along the direction of each coordinate axis. If a difference between the optical waveguides is large, it may be considered that when there is a contact force at the contact point, under the action of the contact force, degrees of squeezing of the contact point on the optical waveguides differ greatly, and consequently, degrees of changes of luminous fluxes of the optical waveguides differ greatly. This usually occurs when the contact force acting on the contact point has a force in a tangential direction. Therefore, if the difference between the optical waveguides is large, it may be considered that the contact force has the force in the tangential direction, and may have a force in a normal direction. A value of the force in the normal direction and a value and a direction of the force in the tangential direction may further be obtained based on a variation of the luminous flux of each optical waveguide and the pre-obtained mapping relationship.

A third aspect of this application provides an electronic device. The electronic device includes a tactile sensing device, and the tactile sensing device is the tactile sensor described in any one of the first aspect or the possible implementations of the first aspect.

In a possible implementation of the third aspect, when the electronic device is a surgical instrument, the electronic device may further include a surgical forceps. The tactile sensing device may be deployed at the end of the surgical forceps, and a contact force sensed by the tactile sensing device is used to indicate the surgical forceps to execute a specified task. In this implementation, the electronic device may further include a display screen, configured to display a value of a force of the contact force in a normal direction and a value of a force in a tangential direction, so that a user can better use the surgical instrument. In this implementation, the electronic device may further include a processor, and the processor executes the specified task by using the contact force sensed by the tactile sensing device.

In a possible implementation of the third aspect, when the electronic device is a mechanical hand, the electronic device further includes a processor. The processor executes a specified task by using a contact force sensed by the tactile sensing device.

In a possible implementation of the third aspect, when the electronic device is a meshworm, the electronic device further includes a processor. The processor executes a specified task by using a contact force sensed by the tactile sensing device.

In a possible implementation of the third aspect, when the electronic device is a steering wheel, the electronic device further includes a handle structure. The tactile sensing device is deployed on a surface of the handle structure, and a processor executes a specified task by using a contact force sensed by the tactile sensing device.

A fourth aspect of this application provides a detection apparatus, including one or more processors. The one or more processors are coupled to a memory, and the memory stores a program. When program instructions stored in the memory are executed by the one or more processors, the method described in any one of the second aspect or the possible implementations of the second aspect is implemented.

A fifth aspect of this application provides a computer-readable storage medium, including a program. When the program is executed by a processing unit, the method described in any one of the second aspect or the possible implementations of the second aspect is performed.

For beneficial effect of the second aspect to the ninth aspect of embodiments of this application, refer to the beneficial effect described in the first aspect. Details are not described again.

**BRIEF DESCRIPTION OF DRAWINGS**

FIG. 1 is a schematic diagram of an application scenario of a tactile sensing device according to an embodiment of this application;
FIG. 2a is a schematic diagram of a principle of a tactile sensor according to an embodiment of this application;
FIG. 2b is a schematic diagram of a principle of a tactile sensor according to an embodiment of this application;
FIG. 2c is a schematic diagram of a principle of a tactile sensor according to an embodiment of this application;
FIG. 3 is a schematic diagram of a structure of a tactile sensor according to an embodiment of this application;
FIG. 4 is a schematic diagram of a structure of another tactile sensor according to an embodiment of this application;
FIG. 5 is a schematic diagram of a structure of another tactile sensor according to an embodiment of this application;
FIG. 6 is a schematic diagram of a structure of another tactile sensor according to an embodiment of this application;
FIG. 7 is a schematic diagram of a structure of another tactile sensor according to an embodiment of this application;
FIG. 8 is a schematic diagram of a structure of another tactile sensor according to an embodiment of this application;
FIG. 9 is a schematic diagram of a structure of another tactile sensor according to an embodiment of this application;
FIG. 10 is a schematic diagram of a structure of another tactile sensor according to an embodiment of this application;
FIG. 11 is a schematic diagram of a structure of another tactile sensor according to an embodiment of this application;
FIG. 12 is a schematic diagram of a structure of another tactile sensor according to an embodiment of this application;
FIG. 13 is a schematic flowchart of a detection method according to an embodiment of this application;
FIG. 14 is a schematic diagram of a principle of a tactile sensor according to an embodiment of this application;
FIG. 15 is a schematic diagram of a structure of another tactile sensor according to an embodiment of this application;
FIG. 16 is a schematic diagram of a structure of another tactile sensor according to an embodiment of this application;
FIG. 17 is a schematic diagram of a structure of another tactile sensor according to an embodiment of this application; and
FIG. 18 is a schematic diagram of a hardware structure of an electronic device according to an embodiment of this application.

**DESCRIPTION OF EMBODIMENTS**

The following describes technical solutions in embodiments of this application with reference to accompanying drawings in embodiments of this application. It is clear that the described embodiments are merely a part rather than all of embodiments of this application. All other embodiments obtained by a person of ordinary skill in the art based on embodiments of this application without creative efforts shall fall within the protection scope of this application.

An embodiment of this application provides a touch sensing device. A plurality of optical waveguides surround a contact point, and change values of luminous fluxes of the plurality of optical waveguides are used to implement decoupling detection on a force acting on the contact point in a tangential direction and a normal direction.

A tactile sensor may also be referred to as the tactile sensing device, and may include one or more tactile sensing units. The tactile sensing unit is configured to sense a contact force acting on a single point. Tactile sensors may be classified into a single-point tactile sensor and a dot-matrix tactile sensor based on a quantity of tactile sensing units. Because the dot-matrix tactile sensor integrates a plurality of tactile sensing units, a contact force may be detected in more scenarios. Specifically, the scenarios may include a scenario in which decoupling detection may be performed on a force acting on the dot-matrix tactile sensor through single contact in a normal direction and a scenario in which decoupling detection may be performed on the force acting on the dot-matrix tactile sensor through single contact in a tangential direction and the normal direction. In this embodiment of this application, a component of a force of the force applied to the contact point along a direction perpendicular to a surface of the contact point is referred to as the force in the normal direction, and a component of a force of the force applied to the contact point along a direction parallel to the surface of the contact point is referred to as the force in the tangential direction.

However, because the dot-matrix tactile sensor needs to rely on the plurality of tactile sensing units to implement decoupling detection on the contact force in the tangential direction and the normal direction, it is difficult to reduce a size of the tactile sensor. With the rapid development of computer technologies, material science, internet technologies, and micromachining means, in increasing application scenarios, the tactile sensor needs to be deployed in a limited region, and the tactile sensor further needs to be able to provide reliable data. To resolve this problem, an embodiment of this application provides a tactile sensor. Each contact point on the tactile sensor can implement decoupling detection on a force acting on the contact point for a single time in a tangential direction and a normal direction. According to the solution provided in this embodiment of this application, external normal force and tangential force perception capabilities are integrated into a single contact point, thereby greatly expanding a dimension of information provided by a single contact point.

The solution provided in this embodiment of this application may be applicable to a plurality of application scenarios, and is particularly applicable to a scenario in which a sensor is deployed in a limited region, and a scenario in which normal force information and tangential force information need to be obtained simultaneously. For example, the following provides description with reference to several typical application scenarios.

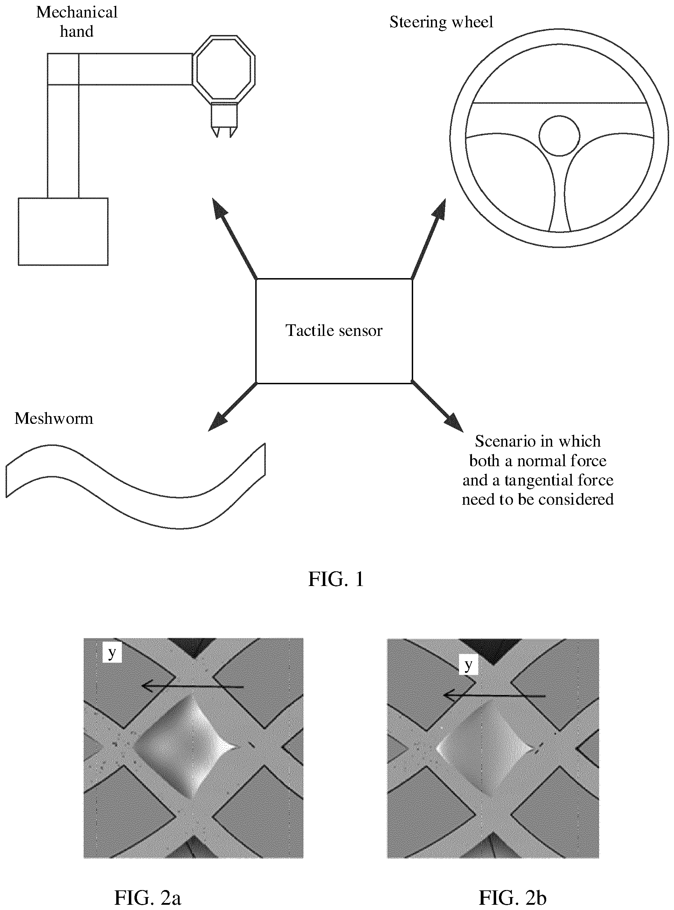

As shown in FIG. 1, in a possible application scenario, the tactile sensor provided in this embodiment of this application may be deployed on a mechanical hand. The mechanical hand is an automatic operating apparatus that can imitate some movement functions of a human hand and arm to grasp or carry an object or operate a tool according to a fixed procedure. In a grasping process, the mechanical hand needs to sense a contact point between the mechanical hand and an object to be grasped, and a value of a force acting on the contact point. According to the tactile sensor provided in this embodiment of this application, the external normal force and tangential force sensing capabilities are integrated into the single contact point, so that the mechanical hand on which the tactile sensor provided in this embodiment of this application is deployed can sense a direction of the force while sensing the value of the force at the contact point. Specifically, a force in a normal direction and a force in a tangential direction may be sensed. A typical application scenario is that when grasping the object, the mechanical hand needs to consider both a normal force and a tangential force to ensure that the object is grasped stably and does not slide off. According to the solution provided in this embodiment of this application, it can be easier for the mechanical hand to determine that the mechanical hand grasps the object by using a most suitable force, to ensure that the object does not slip off and grasping precision of the mechanical hand can be further improved.

In a possible application scenario, the tactile sensor provided in this embodiment of this application may be deployed on a meshworm. The meshworm is a kind of crawling robot, and is widely used in medical field, strong electromagnetic interference field, coal mine field, and the like. The tactile sensor provided in this embodiment of this application may be deployed on the meshworm, and in particular, may be deployed on a miniature meshworm. The meshworm on which the tactile sensor provided in this embodiment of this application is deployed can obtain both a normal force and a tangential force in a crawling process, and can further obtain a friction coefficient of a current crawling environment of the meshworm, to better adjust a crawling speed and flexibly adjust an output power of the meshworm.

In a possible implementation, the tactile sensor provided in this embodiment of this application may be deployed on a surgical instrument. With the continuous development of medical technologies in recent years, a minimally invasive technology, as a new medical technology, can effectively reduce surgical trauma, shorten postoperative time, and increase a surgical success rate. With the foregoing advantages, the minimally invasive technology has been favored by the industry and is considered to be one of most promising medical technology development directions. However, smaller surgical wounds also pose higher technical challenges to doctors. Robots of surgical instruments are being used to perform surgical tasks, and the ends of the surgical instruments are required to be flexible and capable of performing precise operations. For example, when the surgical instrument is a surgical forceps, it is necessary to rely on a tangential force to perform an effective pulling operation. The surgical instrument on which the tactile sensor provided in this embodiment of this application is deployed can better sense a force applied by the end, thereby accurately completing a surgical task.

In a possible implementation, the solution provided in this embodiment of this application may further be deployed on a steering wheel or a seat of a vehicle. How to avoid a driving error of a driver, reduce occurrence of a traffic accident, and improve transportation efficiency of a highway has been a continuous concern in the vehicle field. When the driver turns the steering wheel, a force in a tangential direction and a force in a normal direction are generated simultaneously. The tactile sensing device provided in this embodiment of this application is deployed on the steering wheel, so that a force exerted by the driver on the steering wheel can be better sensed, to determine, based on the force, whether the driver is in a fatigue driving state. Similarly, when the driver sits on the seat, a change in a sitting posture may generate both a force in a tangential direction and a force in a normal direction. The tactile sensor device provided in this embodiment of this application is deployed on the seat, so that a physiological indicator of the driver can be better sensed, thereby providing perception data support for determining whether the driver is the fatigue driving state.

It should be noted that the several application scenarios listed above are not exhaustive examples of possible application scenarios in embodiments of this application. The tactile sensing device provided in this embodiment of this application may be applied to any device that needs to sense tactile information. For example, the tactile sensing device provided in this embodiment of this application may further be deployed on a smart insole. Because a tangential force is a guarantee that a human can normally walk on the ground, whether a motion posture is correct is detected by analyzing a force on the smart insole. The smart insole may be used for rehabilitation treatment, daily training of athletes, shoe design, and the like.

To better understand the solutions provided in embodiments of this application, the following describes a research idea of embodiments of this application.

The optical waveguide is a dielectric apparatus that guides an optical signal to propagate in the dielectric apparatus, and is also known as a dielectric optical waveguide. A principle that the optical waveguide can make the optical signal propagate in the optical waveguide lies in a total reflection phenomenon of the optical signal. When conditions that light enters an optically thinner medium from an optically denser medium and an incident angle is greater than or equal to a critical angle are satisfied, and when a refraction angle is increased to 90 degrees, a refracted light propagates along an interface direction, the incident angle is slightly increased, and the incident light is reflected back to the optically denser medium according to a law of reflection, this phenomenon is referred to as the total reflection phenomenon. Compared with the two media, a medium with a large speed of light (a speed of light in the medium) is referred to as the optically thinner medium, and a medium with a small speed of light is referred to as the optically denser medium. Compared with the optically denser medium, the optically thinner medium has a larger speed of light and a smaller absolute refractive index. The critical angle is calculated by using $C = \arcsin (m/n)$, where $m$ is a refractive index of the optically denser medium, and $n$ is a refractive index of the optically thinner medium.

The optical waveguide includes a waveguide layer and a cladding layer, the cladding layer wraps the waveguide layer, and a refractive index of the waveguide layer is greater than a refractive index of the cladding layer. The cladding layer may directly wrap the waveguide layer, or the cladding layer may indirectly wrap the waveguide layer, in other words, another material or medium may alternatively be included between the cladding layer and the waveguide layer. The cladding layer is equivalent to an optically thinner medium, and the waveguide layer is equivalent to an optically denser medium.

The optical waveguide meets a condition of total reflection. However, when a force is applied to the optical waveguide, or when the optical waveguide is deformed, the total reflection condition of the optical waveguide is destroyed, and the optical signal transmitted in the optical waveguide generates a loss. Therefore, in this embodiment of this application, it is first thought that a relationship may be established between a contact point and the optical waveguide. When a force is applied to the contact point, the optical waveguide is deformed when being subject to the force. The force at the contact point can be sensed by disposing a photoelectric sensing component at an output end of the optical waveguide, and a value of the force at the contact point can be obtained by comparing a variation of a luminous flux obtained by the photoelectric sensing component.

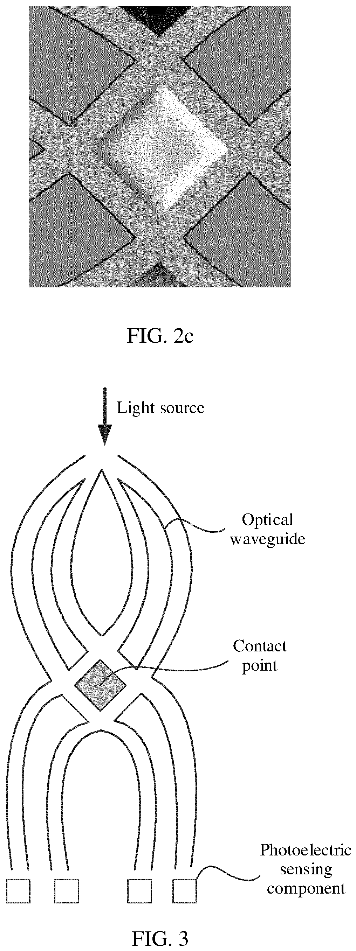

However, in this solution, only a force in one direction can be obtained, and a force in a tangential direction and a force in a normal direction cannot be obtained simultaneously. In other words, this solution cannot implement decoupling detection on a normal force and a tangential force by using a single contact point. In embodiments of this application, it is further thought that the plurality of optical waveguides may be used for cooperation, to achieve this objective. It is found in embodiments of this application that when a plurality of optical waveguides surround one contact point, the contact point is in contact with each optical waveguide. When forces of different directions and different values are acted on the contact point, the plurality of optical waveguides that are in contact with the contact point generate different deformations. It is assumed that four optical waveguides are used to form a quadrangle. When the four optical waveguides surround a contact point, in other words, the contact point is disposed inside the quadrangle, and when no force is applied to the contact point, the contact point is separately in contact with the four optical waveguides. When a force is applied to the contact point, after the force is decomposed, a value of a force along a positive direction of a y axis is 1 N, and a value of a force in a normal direction is 1 N. Deformations of the four optical waveguides are shown in FIG. 2a. When a force is applied to the contact point, after the force is decomposed, a value of a force along a positive direction of a y axis is 1 N, and a value of a force in a normal direction is 0 N. Deformations of the four optical waveguides are shown in FIG. 2b. When a force is applied to the contact point, after the force is decomposed, a value of a force along a positive direction of a y axis is 0 N, and a value of a force in a normal direction is 1 N. Deformations of the four optical waveguides are shown in FIG. 2c. In embodiments of this application, a plane on which a force-bearing surface of the contact point is located is considered as a plane formed by an x-axis and the y-axis, and a z-axis is perpendicular to the plane. Details are not described below again. However, it should be noted that another solution of setting a reference system does not affect implementation of the solution. In embodiments of this application, a coordinate system may alternatively be established in another manner.

Based on this finding, when the forces of different directions and different values are acted on the contact point, the plurality of optical waveguides that surround the contact point and that are in contact with the contact point generate different deformations. With reference to the foregoing description, when the force is applied to the optical waveguide, or when the optical waveguide is deformed, the total reflection condition of the optical waveguide is destroyed, and the optical signal transmitted in the optical waveguide generates the loss. It is further thought in embodiments of this application that a mapping relationship between a variation of a luminous flux of each of the plurality of optical waveguides and the contact force at the contact point may be established. Decoupling detection of the force acting on the single contact point in the normal direction and the tangential direction is implemented based on the variation of the luminous flux of each of the plurality of optical waveguides. In this application, the variation of the luminous flux of the optical waveguide is sometimes described as a variation of a strength of the optical signal transmitted by the optical waveguide, and the two are used to indicate a same meaning. Details are not described below again.

How to implement decoupling detection on the force in the normal direction and the tangential direction based on the value and the direction of the force reflected by the variation of each of the plurality of optical waveguides needs further consideration. In embodiments of this application, it is thought that a force may be decomposed. For example, the force may be decomposed along coordinate axes of the coordinate system. If a component of the force along a direction of each coordinate axis can be reflected by the variation of the luminous flux of each of the plurality of optical waveguides, decoupling detection of the force in the tangential direction and the normal direction can be implemented. A specific coordinate system to be used is not limited in embodiments of this application. For example, the following uses a three-dimensional Cartesian coordinate system as an example for description. Because the three-dimensional Cartesian coordinate system includes three coordinate axes: an x axis, a y axis, and a z axis, components of the force along directions of the three coordinate axes need to be obtained. Theoretically, it is not realistic to use one optical waveguide to reflect information of two dimensions. Therefore, one optical waveguide is used to provide information of one dimension, and variations of luminous fluxes of at least three optical waveguides are required to accurately reflect the components of the force along the directions of the three coordinate axes.

Then, as long as mapping relationships between the variations of the luminous fluxes of the at least three optical waveguides and the components of the force along the directions of the three coordinate axes are found, decoupling detection of the force in the normal direction and the tangential direction can be implemented based on the variations of the luminous fluxes of the at least three optical waveguides and the mapping relationships. The mapping relationships may be obtained in a plurality of manners, for example, in an experiment manner. Specifically, an experiment may be a simulation experiment or a real experiment. For example, in a simulation experiment, first, a visual sensing device in embodiments of this application may be constructed by using mechanical simulation software (for example, solidworks), including constructing a structural relationship between the at least three optical waveguides and a contact point. Then, a component of a force on each coordinate axis is set, and a simulation result may indicate a displacement of a force-bearing location of each optical waveguide under the action of the force. Then, a displacement model obtained after mechanical simulation is imported into optical simulation software (for example, zemax) to perform optical simulation, and a simulation result indicates variations of luminous fluxes after displacements of different force-bearing locations of the at least three optical waveguides generate. The mapping relationships between the variations of the luminous fluxes of the at least three optical waveguides and the components of a force along the directions of the three coordinate axes can be obtained more accurately by using a large quantity of experiments.

Based on the foregoing research idea and with reference to specific implementations, the following specifically describes the tactile sensing device provided in embodiments of this application.

FIG. 3 is a schematic diagram of a structure of a tactile sensing device according to an embodiment of this application. As shown in FIG. 3, the tactile sensing device provided in this embodiment of this application may include a light source, optical waveguides, photoelectric sensing components, and a contact point.

The following separately describes the parts included in the tactile sensing device provided in this embodiment of this application from the following aspects.

1. A plurality of optical waveguides may share the light source, or each optical waveguide may exclusively use a light source.

In a possible implementation, all optical waveguides may share one light source, as shown in the structure shown in FIG. 3. In this implementation, the tactile sensor needs to include only one light source. The structure is simple, which facilitates manufacturing of a tactile sensor with a smaller size, and costs may further be reduced.

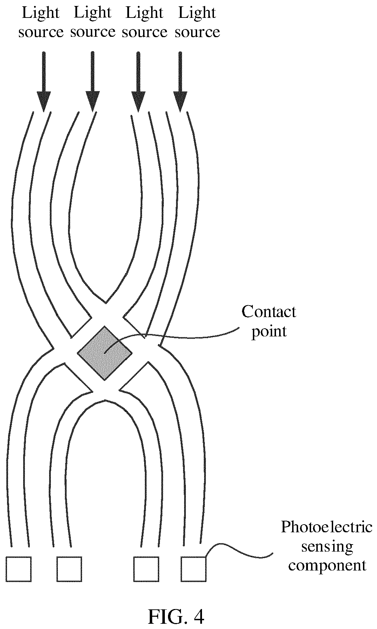

In a possible implementation, each optical waveguide may exclusively use a light source, as shown in a structure shown in FIG. 4. In this implementation, one light source may be disposed at an input end of each optical waveguide. Each optical waveguide exclusively uses one light source, and does not share the light source with other optical waveguides. In this implementation, because each optical waveguide exclusively uses the light source, an optical signal emitted by the light source can be fully absorbed into the optical waveguide, thereby ensuring a strength of the optical signal transmitted in the optical waveguide, and further ensuring that the optical signal emitted by the light source still has a high strength when reaching an intersection of the optical waveguides. This helps to improve detection accuracy.

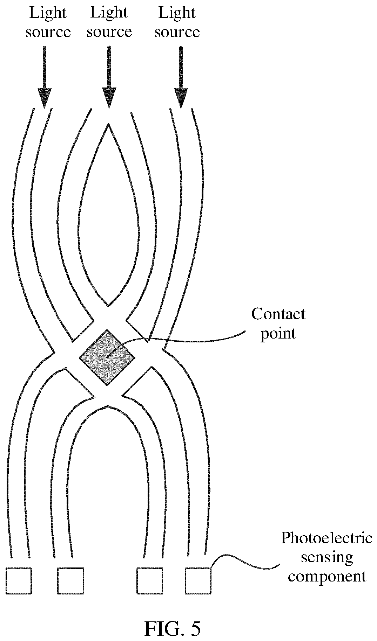

In a possible implementation, based on an actual situation, some optical waveguides may share the light source, and some optical waveguides each exclusively use a light source, as shown in a structure shown in FIG. 5.

To accurately obtain a variation of a luminous flux of each optical waveguide, a photoelectric sensing component needs to be disposed at an output end of each optical waveguide. In other words, there is a one-to-one correspondence between an optical waveguide and a photoelectric sensing component, and one photoelectric sensing component is configured to obtain a strength of an optical signal transmitted by one optical waveguide. The photoelectric sensing component may be any component that converts an optical signal into an electrical signal. For example, in a possible implementation, the photoelectric sensing component may be a photodiode.

Based on the foregoing descriptions, in a possible implementation, the tactile sensor provided in this embodiment of this application may be of a multi-input multi-output structure. In a possible implementation, the tactile sensor provided in this embodiment of this application may alternatively be of a single-input multi-output (single-input multi-output, SIMO) structure.

2. The contact point is in contact with each optical waveguide, and the plurality of optical waveguides surround the contact point.

The contact point is in contact with each optical waveguide, so that each optical waveguide can feel a force acting on the contact point. Specifically, when there is no contact force at the contact point, the contact point can be in contact with each optical waveguide.

It should be noted that, the plurality of optical waveguides surround the contact point, so that when there is a contact force at the contact point, the optical waveguides around the contact point can be deformed accordingly. Further, decoupling detection may be performed on the contact force in a normal direction and a tangential direction based on variations of luminous fluxes of the optical waveguides around the contact point. According to the solution provided in this embodiment of this application, there may be a plurality of structural manners in which the plurality of optical waveguides surround the contact point. In addition, as described in the foregoing research idea, a quantity of optical waveguides in this embodiment of this application is related to a quantity of coordinate axes in a coordinate system used for obtaining a mapping relationship. Specifically, the quantity of optical waveguides cannot be less than the quantity of coordinate axes. This is described below with reference to several preferred structural manners.

In a possible implementation, a shape of the contact point may be a quadrilateral. Refer to shapes of contact points in the structures shown in FIG. 3 to FIG. 5, and each side of the contact point is in contact with one optical waveguide. When a force is applied to the contact point, the contact point squeezes the surrounding optical waveguides, causing the surrounding optical waveguides to deform. As shown in the structures shown in FIG. 3 to FIG. 5, the contact point is fully in contact with each optical waveguide. Specifically, in the structure, each location of each side of the contact point closely adheres to the optical waveguide, or most areas of each side of the contact point closely adheres to the optical waveguide. An advantage of this design is that the optical waveguides around the contact point sufficiently surround the contact point, and the optical waveguides around the contact point can fully feel the force acting on the contact point.

In a possible implementation, the plurality of optical waveguides and the contact point are deployed on a same plane or a same arc surface. In this manner, a size of the tactile sensor may further be reduced. Specifically, a thickness of the tactile sensor can be reduced. For example, the following provides a construction manner:

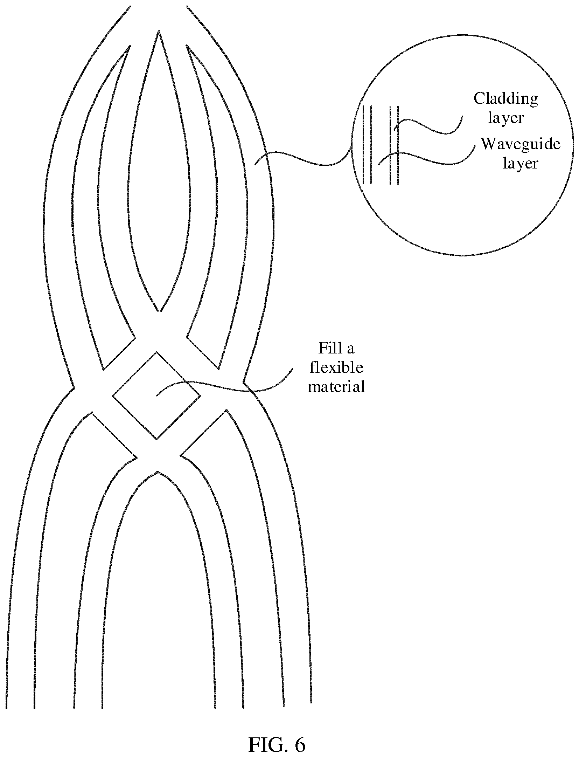

In this implementation, an optical waveguide channel base may be first built. Refer to FIG. 6 for understanding. It is assumed that a tactile sensor having a structure shown in FIG. 6 needs to be constructed. The optical waveguide channel base may be first built by using a material used by a cladding layer based on the structure shown in FIG. 6, where a hard button may be first placed at a location of a contact point to occupy a location. Then, the built optical waveguide channel base is filled with a material used by a waveguide layer. After the to-be-filled material of the optical waveguide is solidified, an outer surface of the optical waveguide may be wrapped with a material of the cladding layer. Finally, the hard button is removed and a material of the contact point is filled, for example, a flexible material. After the flexible material is solidified, the structure that the contact point is surrounded by the plurality of optical waveguides and the contact point is in contact with each optical waveguide is completed. In addition, in this manner, it is ensured that the contact point and the plurality of optical waveguides are deployed on a same plane or a same arc surface, and a thickness of the tactile sensor is reduced.

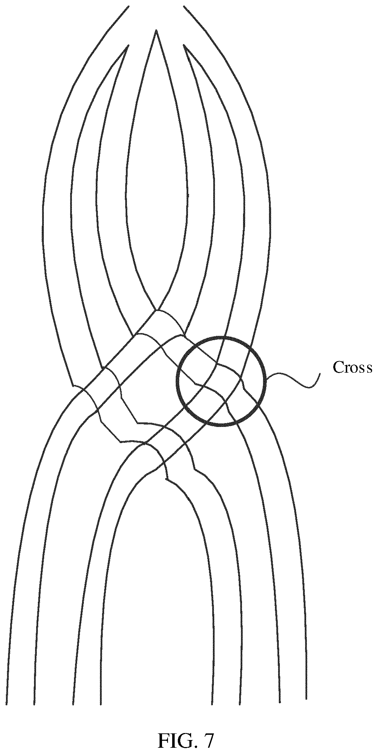

In a possible implementation, the plurality of optical waveguides and the contact point may not be deployed on the same plane or the same arc surface. Different from the solution described with reference to FIG. 6, waveguide layers of the plurality of optical waveguides are shared. In this implementation, each optical waveguide is independent and does not share the waveguide layer with other optical waveguides. For example, refer to FIG. 7 for understanding. Each optical waveguide is independent, and at an intersection of two optical waveguides, one optical waveguide crosses the other optical waveguide. In this implementation, crosstalk between the optical waveguides can be effectively reduced.

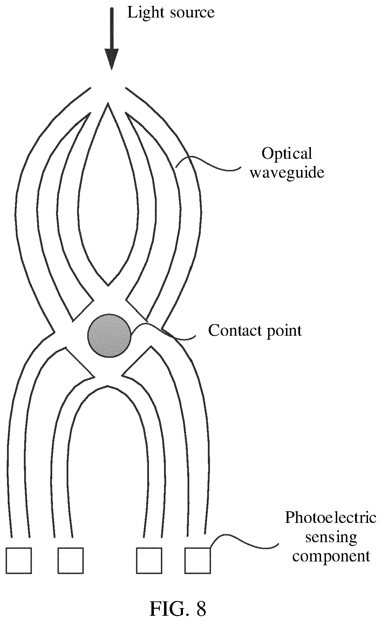

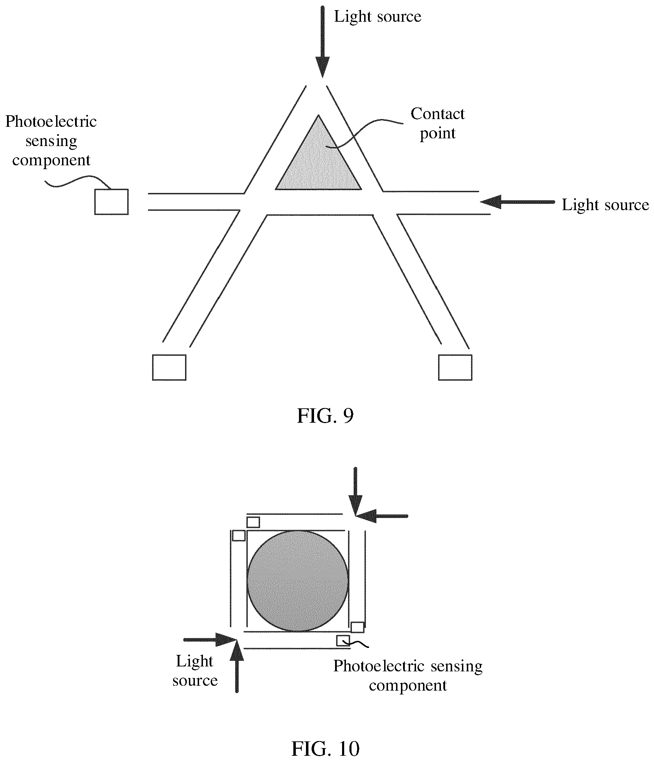

In addition, in addition to designing the contact point as a quadrangle, the contact point may further be designed as another shape. For example, in a structure shown in FIG. 7, to make a profile of the contact point closely adhere to each optical waveguide, the contact point may alternatively be designed as a polygon, to ensure that each side of the contact point can be in contact with one optical waveguide to the maximum extent. For another example, refer to FIG. 8. The shape of the contact point may alternatively be designed as a circle. For another example, refer to FIG. 9. The shape of the contact point may alternatively be a triangle.

In the structures shown in FIG. 3 to FIG. 9, any at least two optical waveguides in the plurality of optical waveguides intersect. An advantage of this design is that light losses can be reduced by increasing a bending radius of the optical waveguide in a plane without increasing an area of the sensor. It should be noted that, in some possible implementations, as long as the plurality of optical waveguides surround the contact point, it is not necessary to meet that the any at least two optical waveguides intersect. Refer to FIG. 10. Any two optical waveguides do not intersect. In addition, a structure shown in FIG. 10 is different from the structures shown in FIG. 3 to FIG. 9 in which the contact point is fully in contact with each optical waveguide. In the structure shown in FIG. 10, the contact point is in contact with a small part of areas of the optical waveguide, or the contact point is tangent to the optical waveguide. A size of a contact region between the contact point and the optical waveguide is not limited in this embodiment of this application. Certainly, a larger contact region between the contact point and the optical waveguide is more conducive to sensing the force acting on the contact point by each optical waveguide, and more conducive to improving sensitivity of the tactile sensor.

In a possible implementation, to reduce interference between optical signals transmitted by different optical waveguides as much as possible, two optical waveguides that intersect may alternatively be designed to be as orthogonal as possible. In other words, a deviation between a degree of an included angle between the two optical waveguides that intersect and 90 degrees is less than a preset threshold, where the preset threshold may be designed to be smaller. In addition, the any two optical waveguides may alternatively be designed not to intersect. Refer to FIG. 10. In the solution provided in this embodiment of this application, the any two optical waveguides do not need to intersect, and it only needs to be ensured that the plurality of optical waveguides surround the contact point. In a possible implementation, it may further be ensured that extension lines of line segments in which the any two optical waveguides are located are perpendicular after they intersect.

In a possible implementation, a radius of a curvature of each optical waveguide should be before a critical value, and the critical value is determined based on a macrobending loss. The macrobending loss is caused when the waveguide layer is bent and the radius of the curvature is before the critical value. An additional optical loss caused by bending is very small and therefore can be ignored. After the critical value, the additional optical loss increases exponentially. In this application, the macrobending loss is considered when the curvature of each optical waveguide is designed, to ensure that the radius of the curvature of each optical waveguide should be before the critical value. In a specific implementation, curvatures of all the optical waveguides may be the same through design, in other words, macrobending losses of all the optical waveguides are the same.

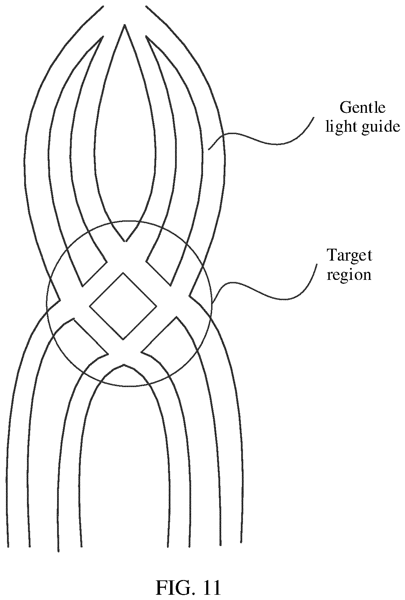

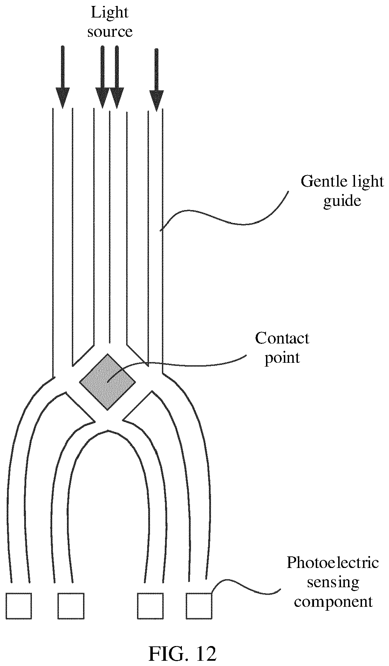

In a possible implementation, to ensure that the optical signal emitted by the light source can still have a sufficient strength when reaching a target region, the optical waveguide may be designed to be a gentle light guide structure from an input end to the target region. For example, in a possible implementation, the optical waveguide may be designed to be an arc-shaped structure from the input end to the target region, and deviations between curvatures of points on an arc-shaped curve are very close, to ensure that the optical signal emitted by the light source can be transmitted to the target region with a few losses after entering the optical waveguide from the input end of the optical waveguide. The reason why the optical signal emitted by the light source can be transmitted to the target region with the few losses is that the curvatures of the points on an arc are so large that macrobending losses of the optical waveguides are small or negligible. Refer to FIG. 11. A range of the target region may be determined based on a range of regions in which the optical waveguides can be deformed accordingly when there is a contact force at the contact point. In a possible implementation, the optical waveguide may be designed to be a linear structure from the input end to the target region. Refer to a structure shown in FIG. 12. Because the optical waveguide uses the linear structure, the optical waveguide basically does not have the macrobending loss. This can ensure to the maximum extent that the optical signal emitted by the light source can be transmitted to the target region with the few losses after entering the optical waveguide from the input end of the optical waveguide. It should be noted that, for different optical waveguides, target regions of all the optical waveguides may be set to be different. In addition, it should be noted that the target region is not an absolute location region. The target region description is introduced in this embodiment of this application to better reflect that the optical waveguide is designed as a gentle light guide shape, so as to ensure that the optical signal emitted by the light source can still have the sufficient strength in a region close to the contact point.

It should be noted that, in different application scenarios, a thickness of each path in the plurality of optical waveguides is adjustable. For example, in some implementations, strengths of optical signals at output ends of one or more paths is required to be stronger, or strengths of optical signals at output ends of one or more paths is required to be weaker. Different requirements may be met by adjusting thicknesses of paths.

In a specific implementation, the tactile sensing device further includes a housing. The light source, the optical waveguides, and the photoelectric sensing components are accommodated in the housing. A target location of the housing is hollowed out, and the target location corresponds to a location of the contact point. In a possible implementation, the housing is a cladding layer of the optical waveguide. In this implementation, a manufacturing process is further simplified, a thickness of the housing of the tactile sensor is reduced, and a function of the cladding layer of the optical waveguide is integrated into the housing of the tactile sensor, thereby simplifying a structure and saving space.

Based on the foregoing descriptions of the structure of the tactile sensor provided in this application, that the tactile sensor may include the light source, the optical waveguides, the photoelectric sensing components, and the contact point is specifically described, and an orientation relationship and a connection relationship between these components are described. The following specifically describes a working principle of the tactile sensor provided in embodiments of this application.

As described above, when a bending radius of the optical waveguide becomes small enough, a propagation angle of light is not suitable for the total reflection condition, and a macrobending loss is generated. In this application, the macrobending loss and a diameter of a path may be used, so that the tactile sensor provided in this application may be disposed on any three-dimensional small curved surface. A microbending loss may be used as the working principle of the tactile sensor in the present invention. To be specific, pressure applied to the contact point causes microbending of the optical waveguide, which also destroys the total reflection condition of the light. Therefore, an optical strength loss generated due to microbending is observed at the output end of the optical waveguide. Specifically, in this solution, the pressure applied to the contact point corresponding to a path causes microbending of the path. At an output end of the path, a light strength loss generated due to microbending can be observed by the photoelectric sensing component. Characteristics of the microbending loss determine a range, sensitivity, and a dynamic response capability of the tactile sensor. The microbending is a place where elastic contact occurs with the outside world, namely, the location of the contact point. A degree of microbending determines the range of the sensor, a light loss degree caused by microbending determines the sensitivity of the sensor, and a recovery speed of microbending after the pressure is removed determines the dynamic response capability of the sensor.

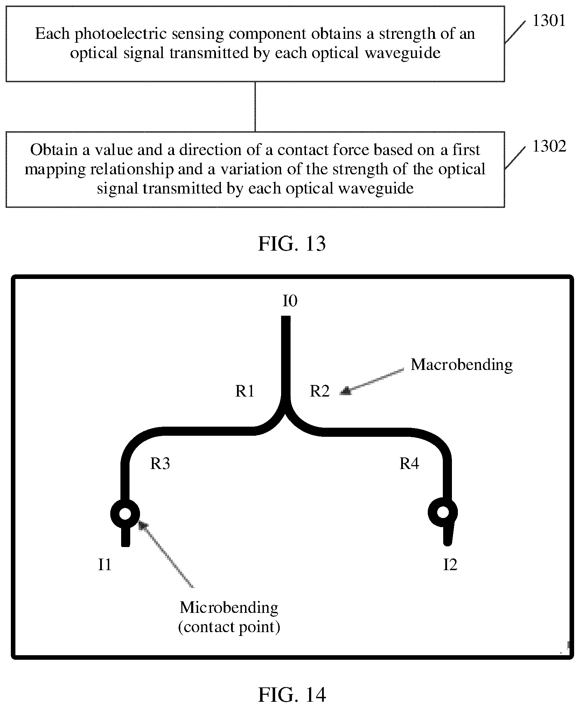

FIG. 13 is a schematic flowchart of a detection method according to an embodiment of this application.

As shown in FIG. 13, the detection method provided in this embodiment of this application may include the following steps.

1301: Each photoelectric sensing component obtains a strength of an optical signal transmitted by each optical waveguide.

The detection method provided in this application is applied to the tactile sensor described in FIG. 1 to FIG. 12. A structure of the tactile sensor is not repeatedly described herein.

The photoelectric sensing component disposed at an output end of each optical waveguide is configured to obtain the strength of the optical signal transmitted by the optical waveguide.

1302: Obtain a value and a direction of a contact force based on a first mapping relationship and a variation of the strength of the optical signal transmitted by each optical waveguide.

It is learned from a large quantity of experiments in this embodiment of this application that, in the tactile sensor described in FIG. 1 to FIG. 12, when the contact force is not greater than a threshold, a component of the contact force along each coordinate axis is linearly related to the variation of the optical signal transmitted by each optical waveguide. Therefore, in this embodiment of this application, a mapping relationship between a value of a force of the contact force along a direction of each coordinate axis and the variation of the strength of the optical signal transmitted by each optical waveguide is obtained by using the large quantity of experiments. When a strength of the optical signal transmitted by each optical waveguide at any moment is obtained, the strength may be compared with a strength of the optical signal transmitted by each optical waveguide in an initial state, to obtain the variation of the light strength. Based on the obtained variation of the light strength and the pre-obtained mapping relationship, decoupling detection can be performed on the contact force at any moment in a tangential direction and a normal direction. In addition, when strengths of the optical signal transmitted by each optical waveguide at any two moments are obtained, a value change trend of the force and a direction change trend of the force between the two moments can be obtained based on changes of the strengths of the optical signal transmitted by each optical waveguide at the any two moments and a mapping relationship.

For example, the following provides a manner in which light strength distribution of each optical waveguide in the initial state (there is no contact force on a contact point) may be determined in advance. It should be noted that, it may be preset that distribution of light strength signals detected by photoelectric sensing components disposed at output ends of all the optical waveguides is consistent when the contact point surrounded by the optical waveguides is not pressed, or it may be preset that distribution of light strength signals detected by photoelectric sensing components disposed at output ends of all the optical waveguides is inconsistent based on an actual requirement when the contact point surrounded by the optical waveguides is not pressed. The following is described by using an example in which it is preset that distribution of the light strength signals detected by the photoelectric sensing components disposed at the output ends of all the optical waveguides is consistent when the contact point surrounded by the optical waveguides is not pressed.

A macrobending loss may be expressed by the following formula:

$$\alpha_c = \frac{1}{2} \frac{\sqrt{\pi}\,U^2}{e_v W^{3/2}\sqrt{aR}V^2 k_{v-1}(W)k_{v+1}(W)} \exp\left(-\frac{2}{3}\frac{W^2}{a^2\beta^2}\frac{R}{a}\right),$$

where

$$\mathrm{U} = \sqrt{k^2 n_1{}^2 - \beta^2},\quad \mathrm{W} = \sqrt{\beta^2 - k^2 n_2{}^2},\ \text{and}\ \ \mathrm{V} = \sqrt{n_1{}^2 - n_2{}^2}\,ka$$

$n_1$ represents a refractive index of a waveguide layer, and $n_2$ is a refractive index of a cladding layer (it should be noted that when a structure whose housing is replaced with the cladding layer, $n_2$ is a refractive index of the housing),

$$\mathrm{k} = \frac{2\pi}{\lambda}$$ is a macrobending spatial frequency, $R$ is a radius of a curvature of optical waveguide bending, $a$ is a radius of the optical waveguide, $\beta$ is a transmission constant, $e_v = 2$ corresponds to a fundamental mode, $e_v = 1$ corresponds to a higher order mode, $k_v$ is a modified Bessel function, and $\lambda$ represents a wavelength of light.

FIG. 14 is a schematic diagram of a principle of a tactile sensor according to an embodiment of this application. As shown in FIG. 14, according to the principle of energy conservation, ideally, energy $I_o$ emitted from a light source is a sum of injection loss energy $I_{in}$, macrobending loss energy $I_{loss}(i)$ of each branch, microbending loss energy $a_m(i)$ of each elastic contact point, and energy $I_r(i)$ received by each photodiode, where the injection loss energy $I_{in}$ is a general term of an "absorption loss" and a "scattering loss". The "absorption loss" is an optical fiber loss caused by an impurity introduced due to an impure material and an imperfect process. The "scattering loss" refers to a loss caused by scattering of light caused by some inhomogeneous materials whose wavelengths are far less than a wavelength. Therefore, the energy emitted by the light source may be expressed by the following formula:

$$I_o = I_{in} + \sum_{i=1}^{2}(I_r(i) + I_{loss}(i) + a_m(i)),$$

where

$$I_{loss}(1) = \alpha_c(R1) + \alpha_c(R3),$$

and

$$I_{loss}(2) = \alpha_c(R2) + \alpha_c(R4)$$

$\alpha_c(R1)$ represents a macrobending loss at R1, $\alpha_c(R3)$ represents a macrobending loss at R3, $\alpha_c(R2)$ represents a macrobending loss at R2, and $\alpha_c(R4)$ represents a macrobending loss at R4.

To achieve light strength equal division of each branch during no contact force, the following formula needs to be satisfied:

$$I_{loss}(i) = I_{loss}(j), i \neq j$$

As shown in FIG. 14, two optical waveguides are used as an example to describe how to make distribution of light strength signals detected by photoelectric sensing components disposed at output ends of all the optical waveguides consistent when a contact point surrounded by the optical waveguides is not pressed. It should be noted that this principle may be extended to a case in which a tactile device includes more optical waveguides.

As described above, in this embodiment of this application, it is learned from the large quantity of experiments that in the tactile sensor described in FIG. 1 to FIG. 12, when the contact force is not greater than the threshold, the component of the contact force along each coordinate axis is linearly related to the variation of the optical signal transmitted by each optical waveguide. The following uses a structure in which four optical waveguides surround a contact point as an example to explain a derivation process of a mapping relationship. When the structure in which the four optical waveguides surround the contact point is used, a mapping relationship between a variation of an optical signal transmitted by each optical waveguide and a value of a force of a contact force along a direction of each coordinate axis may be represented according to the formula $[O]_{4 \times n} = [C]_{4 \times 3} \cdot [F]_{3 \times n}$. $[O]_{4 \times n}$ is used to represent variations of optical signals of the four optical waveguides in a total of n experiments. In a possible implementation, it may be considered that a strength of the optical signal obtained in real time by a photoelectric sensing component disposed at an output end of each optical waveguide is the variation of the optical signal. where $[F]_{3 \times n}$ is used to represent components of a force along an x axis,

$$[F]_{3 \times 1} = \begin{bmatrix} F_x \\ F_y \\ F_z \end{bmatrix}$$

a y axis, and a z axis in the total of n experiments. For example, when n is 1, there is $[F]_{3 \times 1} = \begin{bmatrix} F_x \\ F_y \\ F_z \end{bmatrix}$. $[C]_{4 \times 3}$ is used to represent mapping relationships between the variations of the optical signals of the four optical waveguides or the strengths of the optical signals transmitted by the four optical waveguides and a value of a force along the direction of each coordinate axis. In this case, $[C]_{4 \times 3} = [O]_{4 \times n} \cdot [F]^{-1}_{n \times 3}$, or $[C]_{4 \times 3} = [O]_{4 \times n} \cdot [F]^{-1}_{n \times 3}$ may be expressed as $[C]_{4 \times 3}$

= $[O]_{4\times n} \cdot ([F]^T_{n\times 3} \cdot [F]_{3\times n})^{-1} \cdot [F]^T_{n\times 3}$. For example, refer to Table 1. A group of real experimental data is provided. An experimental process is to build the structure shown in FIG. 3 by using simulation software solidworks. Then, a component of a force on each coordinate axis is set (for the component of the force set on each coordinate axis in a specific experiment process, refer to Table 1). A simulation result may indicate a displacement of a force-bearing location of each optical waveguide under the action of the force. Then, a displacement model after mechanical simulation is imported into optical simulation software zemax for optical simulation, and a simulation result indicates variations of luminous fluxes after the displacements of the four optical waveguides at different force-bearing locations. Strengths of optical signals transmitted by the optical waveguides obtained in the experimental process is shown in Table 1. In this experiment, 12 groups of tests are performed in total. $[C]_{4\times 3}$ can be obtained by substituting $[F]_{3\times n}$ set in the 12 groups of tests and $[O]_{4\times n}$ correspondingly obtained into the foregoing formula $[C]_{4\times 3} = [C]_{4\times n} \cdot [F]^{-1}_{3\times n}$. Alternatively, $[C]_{4\times 3}$ can be obtained by substituting $[F]_{3\times n}$ set in the 12 groups of tests and $[O]_{4\times n}$ correspondingly obtained into the foregoing formula $[C]_{4\times 3} = [O]_{4\times n} \cdot ([F]^T_{n\times 3} \cdot [F]_{3\times n})^{-1} \cdot [F]^T_{n\times 3}$. So far, calculation of obtaining the mapping relationship is completed. When any contact force is obtained again, components of the force along the x axis, the y axis, and the z axis may be obtained based on the strength of the optical signal obtained by each photoelectric sensing component and $[C]_{4\times 3}$ obtained in advance, that is, $\begin{bmatrix} F_x \\ F_y \\ F_z \end{bmatrix}$. A value and a direction of the force in a tangential direction can be obtained based on $F_x$ and $F_y$, and a value of the force in a normal direction can be obtained based on $F_z$.

Table 1

| Channel 1 (mW) | Channel 2 (mW) | Channel 3 (mW) | Channel 4 (mW) | Tangential force along an x-axis direction (N) | Tangential force along a y-axis direction (N) | Normal force (N) |
|---|---|---|---|---|---|---|
| 0.17335 | 0.086825 | 0.086825 | 0.17405 | 0 | 1 | 1 |
| 0.18779 | 0.0819 | 0.0819 | 0.17402 | -0.70711 | 0.707107 | 0.5 |
| 0.19432 | 0.080645 | 0.080645 | 0.18329 | -0.5 | 0 | 1 |
| 0.19648 | 0.07486 | 0.07486 | 0.19005 | -0.70711 | -0.70711 | 1 |
| 0.1984 | 0.084455 | 0.084455 | 0.19887 | 0 | -1 | 0.5 |
| 0.19963 | 0.078305 | 0.078305 | 0.19427 | -0.70711 | -0.70711 | 0.5 |
| 0.19601 | 0.081 | 0.081 | 0.18419 | -0.7 | 0 | 0.5 |
| 0.1846 | 0.078365 | 0.078365 | 0.16812 | -0.70711 | 0.707107 | 1 |
| 0.1769 | 0.087305 | 0.087305 | 0.18327 | 0 | 0.54 | 0.76 |
| 0.19782 | 0.081325 | 0.081325 | 0.19271 | -0.35355 | -0.35355 | 1 |
| 0.1983 | 0.08266 | 0.08266 | 0.19812 | 0 | -1 | 1 |
| 0.17586 | 0.088185 | 0.088185 | 0.18349 | 0 | 0.5 | 1 |

The foregoing describes a core structure of the tactile sensor and the working principle of the tactile sensor provided in embodiments of this application. According to the tactile sensor provided in embodiments of this application, a single contact point can be used to implement decoupling detection on a force in a normal direction and a tangential direction. In an actual application scenario of this solution, more contact points may be deployed on the tactile sensor. This is not limited in this embodiment of this application. Each of a plurality of contact points can be used to implement decoupling detection on a force in a normal direction and a tangential direction. In addition, in some scenarios, only the force in the tangential direction needs to be detected. For these scenarios, the structure of the tactile sensor provided in this embodiment of this application may further be simplified. The following describes these scenarios with reference to some specific embodiments.

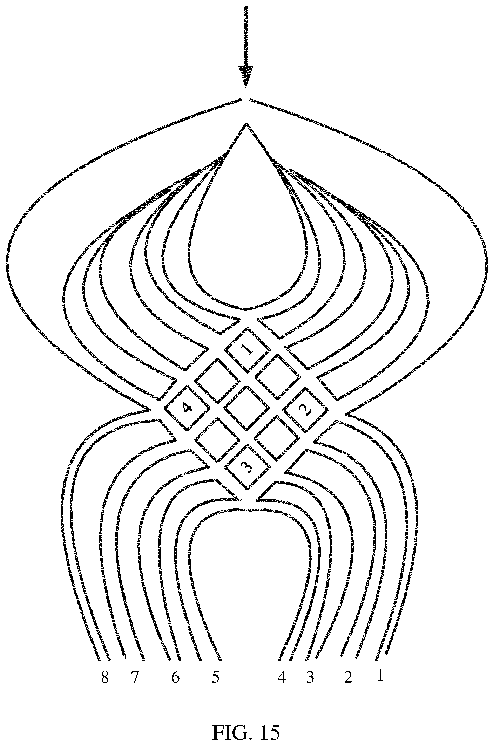

FIG. 15 is a schematic diagram of a structure of another tactile sensor according to an embodiment of this application. In this implementation, more contact points are deployed on the tactile sensor, and each contact point may be used to implement decoupling detection on a contact force applied to the contact point in a tangential direction and a normal direction. When the more contact points are deployed on the tactile sensor, a structure of each contact point on the tactile sensor may be understood with reference to a structure of a single contact point described in FIG. 3 to FIG. 12. For example, the following describes the tactile sensor in which the more contact points are deployed by using four contact points included in the tactile sensor shown in FIG. 15 as an example. The tactile sensor shown in FIG. 15 includes a contact point 1, a contact point 2, a contact point 3, and a contact point 4. In the tactile sensor shown in FIG. 15, the structure described in FIG. 3 is used for a structure of each contact point. Specifically, the contact point 1 is surrounded by an optical waveguide 8, an optical waveguide 7, an optical waveguide 2, and an optical waveguide 1. The contact point 2 is surrounded by an optical waveguide 6, an optical waveguide 5, the optical waveguide 2, and the optical waveguide 1. The contact point 3 is surrounded by the optical waveguide 6, the optical waveguide 5, an optical waveguide 4, and an optical waveguide 3. The contact point 4 is surrounded by the optical waveguide 8, the optical waveguide 7, the optical waveguide 4, and the optical waveguide 3. In this implementation, because optical waveguides surrounding two contact points are not exactly the same, when four photoelectric sensing components in eight photoelectric sensing components disposed at output ends of the optical waveguides learn that strengths of optical signals change, it may be determined that there is a contact force on a contact point surrounded by the four optical waveguides. Further, decoupling detection can be performed on a force at the contact point based on the strengths of the optical signals transmitted by the four optical waveguides and pre-obtained mapping relationships. For example, it is assumed that strengths of optical signals transmitted in a photoelectric sensing component 8, a photoelectric sensing component 7, a photoelectric sensing component 2, and a photoelectric sensing component 1 of the eight photoelectric sensing components change. The photoelectric sensing component 8 is disposed at an output end of the optical waveguide 8, the photoelectric sensing component 7 is disposed at an output end of the optical waveguide 7, the photoelectric sensing component 2 is disposed at an output end of the optical waveguide 2, and the photoelectric sensing component 1 is disposed at an output end of the optical waveguide 1. Therefore, it can be determined that the optical waveguide 8, the optical waveguide 7, the optical waveguide 2, and the optical waveguide 1 are deformed, and it can be further determined that there is a contact force at the contact point 1 surrounded by the optical waveguide 8, the optical waveguide 7, the optical waveguide 2, and the optical waveguide 1. Therefore, decoupling detection of the contact force at the contact point 1 in a tangential direction and a normal direction can be performed based on variations of the strengths of the optical signals of the optical waveguide 8, the optical waveguide 7, the optical waveguide 2, and the optical waveguide 1 and mapping relationships. Specifically, decoupling detection of the contact force at the contact point 1 is performed in the tangential direction and the normal direction based on the strengths of the optical signals obtained by the photoelectric sensing component 8, the photoelectric sensing component 7, the photoelectric sensing component 2, and the photoelectric sensing component 1 and the mapping relationships.

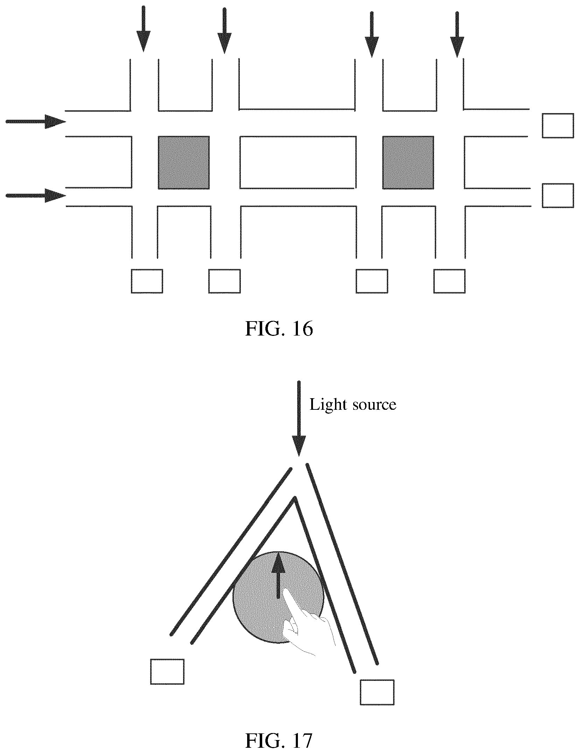

It should be noted that the structure shown in FIG. 15 is merely used to illustrate that more contact points may be deployed on the tactile sensor. A structure of each contact point can also be understood with reference to the structure of the single contact point described in FIG. 3 to FIG. 12. To better understand a structure in which more contact points are deployed, this embodiment of this application further provides a possible structure of a tactile sensor in which more contact points are deployed and that is shown in FIG. 16. In addition, it should be noted that, in the structures of the multi-contact-point tactile sensor shown in FIG. 15 and FIG. 16, there is a repetition of optical waveguides surrounding contact points. This does not mean that in the structures of the multi-contact-point tactile sensor provided in embodiments of this application, there needs to be a repetition between optical waveguides of contact points. In some possible implementations, in the multi-contact-point tactile sensor, there may be no repetition between optical waveguides surrounding contact points. An advantage of this design is that a plurality of contact points on the tactile sensor may be pressed simultaneously. At the same time, decoupling detection of contact forces on the plurality of contact points is implemented. The advantage of such a design with the repetition between the optical waveguides surrounding the contact points is that it is possible to simplify the structure of the tactile sensor and help to reduce a size of the tactile sensor.

In some possible implementations, only a force in the tangential direction needs to be detected. For these scenarios, the structure of the tactile sensor provided in this embodiment of this application may further be simplified. In the tactile sensor described above in FIG. 3 to FIG. 12, a plurality of optical waveguides completely surround a contact point. Further, the contact point is pressed by using forces in different directions. The optical waveguides surrounding the contact point generate different deformations, so that strengths of optical signals transmitted by the plurality of optical waveguides change differently. Further, the tactile sensor can implement decoupling detection on the force in a normal direction and a tangential direction. In addition, it is further mentioned above that it is not realistic to use one optical waveguide to reflect information of two dimensions. Therefore, one optical waveguide is used to provide information of one dimension, and variations of luminous fluxes of at least three optical waveguides are required to accurately reflect components of a force along directions of three coordinate axes. Based on these considerations, if the force is detected in the tangential direction only by photoelectric sensing components, variations of luminous fluxes of two optical waveguides can be used to reflect components of the force along directions of two coordinate axes. If a z-axis is used to indicate the normal direction, the tangential force can be detected, including obtaining a value and a direction of the tangential force, by reflecting components in x-axis and y-axis directions based on the variations of the luminous fluxes of the two optical waveguides. This is described below with reference to a specific embodiment. FIG. 17 is a schematic diagram of a structure of another tactile sensor according to an embodiment of this application. Similar to working principles of the tactile sensor described in FIG. 3 to FIG. 12, in the structure shown in FIG. 17, a contact point is also in contact with each of two optical waveguides. The working principle of the tactile sensor in this embodiment of this application is that the optical waveguides surrounding the contact point generate different deformations by applying forces of different values and different directions to the contact point. When the two optical waveguides are used, and boundaries at which the two optical waveguides are in contact with the contact point are parallel, it is difficult to ensure that the two optical waveguides can generate different deformations by applying the forces of different values and different directions. Therefore, to enable the tactile sensor to use the two optical waveguides, and to fully satisfy the working principle of the tactile sensor, the boundaries at which the two optical waveguides are in contact with the contact point may be deployed as non-parallel to each other. In a possible implementation, the two optical waveguides may be alternatively deployed as crossed. In addition, because the used two optical waveguides cannot sufficiently surround the contact point as the structure described in FIG. 3 to FIG. 12, an inaccurate test result may occur. For example, in the structure shown in FIG. 17, when a tangential force in a positive direction of a y axis is applied to the contact point, the contact point may sufficiently squeeze the two optical waveguides under the action of the force. If a tangential force in a negative direction of the y axis is applied to the contact point, the two optical waveguides may not be fully squeezed, and changes in luminous fluxes of the two optical waveguides are not obvious, thereby affecting a test result. Explanation is provided from another perspective. In this embodiment of this application, because a force is detected by using a variation of a luminous flux of an optical waveguide and a contact point is not completely surrounded, components of the force along some directions are not fed back by luminous fluxes corresponding to optical waveguides. As a result, two tangential forces may be finally obtained based on pre-obtained mapping relationships and variations of the luminous fluxes of the two optical waveguides, and which tangential force causes the problem cannot be accurately determined. Therefore, to resolve this problem, the contact point may further be preset to be bound to a coordinate axis direction. For example, the contact point is bound to the positive direction of the y axis. When the variation of the luminous flux of the optical waveguide is obtained, it is determined that the component of the force on the y axis can be only a component along the positive direction of the y axis, and cannot be a component along the negative direction of the y axis.

In some possible implementations, only whether a force in a tangential direction or a force in a normal direction exists on the tactile sensor may need to be obtained. In the solution provided in this embodiment of this application, these scenarios may further be determined based on a difference between luminous fluxes of optical waveguides. For example, in a possible implementation, if the difference between the optical waveguides is small, it may be considered that when there is a contact force at the contact point, under the action of the contact force, degrees of squeezing of the contact point on the optical waveguides are similar, and consequently, degrees of changes of the luminous fluxes of the optical waveguides are similar. This usually occurs when the contact force acting on the contact point has a force only in a normal direction, and does not have a force in a tangential direction. Therefore, if the difference between the optical waveguides is small, it may be considered that the contact force is the force in the normal direction. In addition, in this case, the force along the z-axis direction may be obtained based on the variation of the luminous flux of each optical waveguide and a pre-obtained mapping relationship, and the forces along the x-axis and y-axis directions may not be obtained, thereby reducing a calculation amount. On the contrary, in a possible implementation, if the difference between the optical waveguides is large, it may be considered that when there is a contact force at the contact point, under the action of the contact force, degrees of squeezing of the contact point on the optical waveguides differ greatly, and consequently, degrees of changes of the luminous fluxes of the optical waveguides differ greatly. This usually occurs when the contact force acting on the contact point has a force in a tangential direction. Therefore, if the difference between the optical waveguides is small, it may be considered that the contact force has the force in the tangential direction, and may have a force in a normal direction. A value of the force in the normal direction, and a value and a direction of the force in the tangential direction may further be obtained based on the variation of the luminous flux of each optical waveguide and the pre-obtained mapping relationship.

This application further provides an electronic device. The electronic device includes a processor and a tactile sensor. The tactile sensor is the tactile sensor described in FIG. 3 to FIG. 17. The electronic device provided in this application may be any device that needs to be configured with the tactile sensor, for example, a mobile phone, a watch, a headset, a computer, a mechanical hand, a meshworm, a steering wheel, or a smart home.

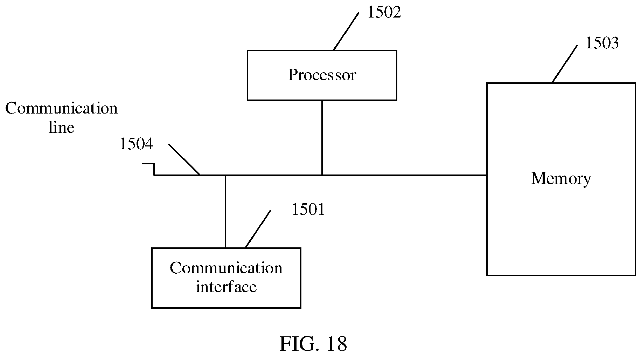

For example, the electronic device provided in this application may be implemented by using a structure shown in FIG. 18. FIG. 18 is a schematic diagram of a hardware structure of an electronic device according to an embodiment of this application. The electronic device includes a communication interface 1501 and a processor 1502, and may further include a memory 1503. It should be noted that structures included in the electronic device listed herein are merely examples for description. In an actual application scenario, more or fewer components may be included, for example, a display screen may further be included.

The communication interface 1501 may use any apparatus such as a transceiver, and is configured to communicate with another device or a communication network.

The processor 1502 includes but is not limited to one or more of a central processing unit (central processing unit, CPU), a network processor (network processor, NP), an application-specific integrated circuit (application-specific integrated circuit, ASIC), or a programmable logic device (programmable logic device, PLD). The PLD may be a complex programmable logic device (complex programmable logic device, CPLD), a field-programmable gate array (field-programmable gate array, FPGA), generic array logic (generic array logic, GAL), or any combination thereof. The processor 1502 is responsible for a communication line 1504 and general processing, and may further provide various functions, including timing, a peripheral interface, voltage regulation, power management, and other control functions. The memory 1503 may be configured to store data used when the processor 1502 performs an operation.

The memory 1503 may be a read-only memory (read-only memory, ROM) or another type of static storage device that can store static information and instructions, or a random access memory (random access memory, RAM) or another type of dynamic storage device that can store information and instructions. Alternatively, the memory 1503 may be an electrically erasable programmable read-only memory (electrically erasable programmable read-only memory, EEPROM), a compact disc read-only memory (compact disc read-only memory, CD-ROM) or another compact disc storage, an optical disc storage (including a compact disc, a laser disc, an optical disc, a digital versatile disc, a Blu-ray optical disc, and the like), a magnetic disk storage medium or another magnetic storage device, or any other medium that can be used to carry or store expected program code in a form of an instruction or a data structure and that can be accessed by a computer. However, the memory 1503 is not limited thereto. The memory may exist independently, and is connected to the processor 1502 through the communication line 1504. Alternatively, the memory 1503 may be integrated into the processor 1502. If the memory 1503 and the processor 1502 are mutually independent components, the memory 1503 is connected to the processor 1502. For example, the memory 1503 and the processor 1502 may communicate through the communication line. The communication interface 1501 and the processor 1502 may communicate with each other through the communication line, or the communication interface 1501 may be directly connected to the processor 1502.

The communication line 1504 may include any quantity of interconnected buses and bridges, and the communication line 1504 links together various circuits including the one or more processors 1502 represented by the processor 1502 and the memory represented by the memory 1503. The communication line 1504 may further link various other circuits such as a peripheral device, a voltage stabilizer, and a power management circuit. These circuits are well known in the art, and therefore are not further described in this application.

In a specific implementation, the electronic device further includes a memory and a processor. The memory and the processor are coupled, the memory includes instructions, and the processor executes a specified task by using a contact force sensed by a tactile sensing device and the instructions. For example, in a possible implementation, when the electronic device is a surgical instrument, a force for pulling a surgical wire may be adjusted based on a tangential force and a normal force that are sensed by the tactile sensing device and the instructions stored in the memory. For another example, in a possible implementation, when the electronic device is a meshworm, a crawling speed may be adjusted based on a tangential force and a normal force that are sensed by the tactile sensing device and the instructions stored in the memory, to flexibly adjust an output power of the meshworm. For another example, in a possible implementation, when the electronic device is a steering wheel of a vehicle, whether a driver is in a fatigue driving state may be determined based on a tangential force and a normal force that are sensed by the tactile sensing device and the instructions stored in the memory.

For the detection method provided in embodiments of this application, a person of ordinary skill in the art may understand that all or some of the steps of the detection method may be implemented by hardware, or may be implemented by a program instructing related hardware. The program may be stored in a computer-readable storage medium. The storage medium mentioned above may be a read-only memory, a magnetic disk, an optical disc, or the like. A person skilled in the art may clearly understand that, in this application, the detection method provided in embodiments of this application may be implemented by using software in addition to necessary universal hardware. Certainly, the detection method provided in embodiments of this application may alternatively be implemented by using dedicated hardware including an application-specific integrated circuit, a dedicated CPU, a dedicated memory, a dedicated component, and the like. Generally, any function that can be performed by a computer program can be easily implemented by using corresponding hardware. Moreover, a specific hardware structure used to achieve a same function may be in various forms, for example, in a form of an analog circuit, a digital circuit, or a dedicated circuit. However, as for this application, software program implementation is a better implementation in most cases. Based on such an understanding, the technical solutions of this application essentially or the part contributing to the conventional technology may be implemented in a form of a software product. The computer software product is stored in a readable storage medium, for example, a floppy disk, a USB flash drive, a removable hard disk, a read-only memory ROM, a random access memory RAM, a magnetic disk, or an optical disc of a computer, and includes several instructions for instructing a computer device (which may be a personal computer, a server, a network device, or the like) to perform the detection method provided in embodiments of this application.

All or some of the detection methods in embodiments of this application may be implemented by using software, hardware, firmware, or any combination thereof. When software is used to implement the detection methods, all or a part of the detection methods may be implemented in a form of a computer program product.

The computer program product includes one or more computer instructions. When the computer program instructions are loaded and executed on a computer, all or some of the procedures or functions described in the detection method provided in embodiments of this application are generated. The computer may be a general-purpose computer, a special-purpose computer, a computer network, or another programmable device. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state disk (solid-state disk, SSD)), or the like.

In the specification, claims, and accompanying drawings of this application, the terms "first", "second", "third", "fourth", and so on (if existent) are intended to distinguish between similar objects but do not necessarily indicate a specific order or sequence. It should be understood that the data termed in such a way is interchangeable in proper circumstances so that embodiments described herein can be implemented in other orders than the order illustrated or described herein. In addition, the terms "include" and "have" and any other variants are intended to cover the nonexclusive inclusion. For example, a process, method, system, product, or device that includes a list of steps or units is not necessarily limited to those expressly listed steps or units, but may include other steps or units not expressly listed or inherent to such a process, method, product, or device.

Finally, it should be noted that the foregoing descriptions are merely specific implementations of this application, but are not intended to limit the protection scope of this application. The protection scope of this application shall be subject to the protection scope of the claims.

**Claims**

1. A tactile sensing device, comprising a light source, a plurality of optical waveguides, photoelectric sensing components, and a contact point, wherein
   the light source is disposed at an input end of each optical waveguide, the photoelectric sensing component is disposed at an output end of each optical waveguide, the plurality of optical waveguides surround the contact point, the contact point is in contact with each optical waveguide, the plurality of optical waveguides are deformed when there is a contact force at the contact point, the photoelectric sensing component obtains an optical signal transmitted by each optical waveguide, and a variation of the optical signal transmitted by each optical waveguide is used to detect the contact force.

2. The tactile sensing device according to claim 1, wherein the contact point is polygonal, and each side of the contact point is in contact with one of the optical waveguides.

3. The tactile sensing device according to claim 1, wherein a profile of the contact point is arc-shaped.

4. The tactile sensing device according to any one of claims 1 to 3, wherein a region between an input end of the optical waveguide and a contact location is an arc-shaped structure, and the contact location is a location in which the optical waveguide is in contact with the contact point.

5. The tactile sensing device according to claim 4, wherein a curvature of the arc is less than a first preset threshold.

6. The tactile sensing device according to any one of claims 1 to 3, wherein a region between an input end of the optical waveguide and a contact location is a linear structure, and the contact location is a location in which the optical waveguide is in contact with the contact point.

7. The tactile sensing device according to any one of claims 1 to 6, wherein any at least two optical waveguides in the plurality of optical waveguides intersect.

8. The tactile sensing device according to claim 7, wherein a deviation between a degree of an included angle between two optical waveguides that intersect and 90 degrees is less than a second preset threshold.

9. The tactile sensing device according to any one of claims 1 to 8, wherein the plurality of optical waveguides are deployed on a same reference plane.

10. The tactile sensing device according to claim 9, wherein the contact point and the plurality of optical waveguides are deployed on the same reference plane.

11. The tactile sensing device according to any one of claims 1 to 10, wherein the plurality of optical waveguides are of multiple-input multiple-output structures.

12. The tactile sensing device according to any one of claims 1 to 10, wherein at least two optical waveguides in the plurality of optical waveguides are of single-input multiple-output SIMO structures, and one light source is disposed at an input end of the at least two optical waveguides with the same input end.

13. The tactile sensing device according to any one of claims 1 to 12, wherein the tactile sensing device further comprises a housing, the light source, the plurality of optical waveguides, and the photoelectric sensing component are accommodated in the housing, a target location of the housing is hollowed out, and the target location corresponds to a location of the contact point.

14. The tactile sensing device according to any one of claims 1 to 13, wherein when there is no contact force at the contact point, a deviation of macrobending losses of any two optical waveguides falls within a preset range.

15. The tactile sensing device according to any one of claims 1 to 14, wherein a quantity of contact points is not less than 2.

16. An electronic device, wherein the electronic device comprises a tactile sensing device, and the tactile sensing device is the tactile sensing device described in any one of claims 1 to 15.

17. The electronic device according to claim 16, wherein when the electronic device is a surgical instrument, the electronic device further comprises a surgical forceps; the tactile sensing device is disposed at the end of the surgical forceps; and a contact force sensed by the tactile sensing device is used to indicate the surgical forceps to execute a specified task.

18. The electronic device according to claim 16, wherein when the electronic device is a mechanical hand, the electronic device further comprises a processor; and the processor executes a specified task by using a contact force sensed by the tactile sensing device.

19. The electronic device according to claim 16, wherein when the electronic device is a meshworm, the electronic device further comprises a processor; and the processor executes a specified task by using a contact force sensed by the tactile sensing device.

20. The electronic device according to claim 16, wherein when the electronic device is a steering wheel, the electronic device further comprises a handle structure; the tactile sensing device is deployed on a surface of the handle structure; and the processor executes a specified task by using a contact force sensed by the tactile sensing device.

21. A detection method, wherein the detection method is applied to a tactile sensor, the tactile sensor is the tactile sensing device described in any one of claims 1 to 15, and the method comprises:

obtaining, by a photoelectric sensing component, a strength of an optical signal transmitted by each optical waveguide; and
obtaining a value and a direction of the contact force based on a first mapping relationship and a variation of the strength of the optical signal transmitted by each optical waveguide, wherein the first mapping relationship comprises mapping between a value of a force of the contact force along a direction of each coordinate axis and the variation of the strength of the optical signal transmitted by each optical waveguide.

22. The detection method according to claim 21, wherein the obtaining a value and a direction of the contact force based on a first mapping relationship and a variation of the strength of the optical signal transmitted by each optical waveguide comprises:

when a deviation between a first variation and a second variation falls within a preset range, determining that the direction of the contact force is downward and perpendicular to a plane on which the contact point is located, wherein the first variation and the second variation are variations of strengths of optical signals transmitted by any two optical waveguides; and

obtaining the value of the contact force based on an absolute value of the first variation, an absolute value of the second variation, and the first mapping relationship.

23. The detection method according to claim 21, wherein the obtaining a value and a direction of the contact force based on a first mapping relationship and a variation of the strength of the optical signal transmitted by each optical waveguide comprises:

when a deviation between a first variation and a second variation does not fall within a preset range, obtaining the value of the force of the contact force along the direction of each coordinate axis based on the first mapping relationship, the first variation, and the second variation, wherein the first variation and the second variation are variations of strengths of optical signals transmitted by any two optical waveguides; and

obtaining the value and the direction of the contact force based on the value of the force of the contact force along the direction of each coordinate axis.

Mechanical
hand

Steering wheel

Tactile sensor

Meshworm

Scenario in which
both a normal force
and a tangential force
need to be considered

FIG. 1

y

y

FIG. 2a

FIG. 2b

FIG. 2c

Light source

Optical
waveguide

Contact
point

Photoelectric
sensing
component

FIG. 3

Light Light Light Light
source source source source

Contact
point

Photoelectric
sensing
component

FIG. 4

Light    Light    Light
source  source  source

Contact
point

Photoelectric
sensing
component

FIG. 5

Cladding
layer

Waveguide
layer

Fill a
flexible
material

FIG. 6

Cross

FIG. 7

Light source

Optical
waveguide

Contact point

Photoelectric
sensing
component

FIG. 8

FIG. 9

FIG. 10

Gentle
light guide

Target
region

FIG. 11

FIG. 12

Each photoelectric sensing component obtains a strength of an optical signal transmitted by each optical waveguide  / 1301

Obtain a value and a direction of a contact force based on a first mapping relationship and a variation of the strength of the optical signal transmitted by each optical waveguide  / 1302

FIG. 13

I0

R1        R2        Macrobending

R3                              R4

I1                              I2

Microbending
(contact point)

FIG. 14

FIG. 15

FIG. 16

Light source

FIG. 17

1502

Processor

1503

Communication
line

1504

Memory

1501

Communication
interface

FIG. 18

## INTERNATIONAL SEARCH REPORT

| International application No. |
|---|
| **PCT/CN2021/113908** |

| **A.** | **CLASSIFICATION OF SUBJECT MATTER** |
|---|---|

G01L 1/24(2006.01)i

According to International Patent Classification (IPC) or to both national classification and IPC

| **B.** | **FIELDS SEARCHED** |
|---|---|

Minimum documentation searched (classification system followed by classification symbols)

G01L, G01B

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 切向, 切线, 剪切, 法向, 多维, 三维, 三轴, 多轴, 力, 触点, 触觉, 传感, 光波导, 光纤, 光学纤维, 光缆, 通道, 变形, 形变, shear, normal, multi+, 3D, dimension+, three, tri+, axis, force, tactile, sens+, contact+, optic +, waveguide?, channel+, deform+

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | JAMIL, Babar et al. ""Force Sensing Fingertip with Soft Optical Waveguides for Robotic Hands and Grippers"" <br> *2018 IEEE International Conference on Soft Robotics (RoboSoft),* 09 July 2018 (2018-07-09), page 148, left column, last paragraph to page 151, left column, last paragraph, and figures 2-11 | 1-23 |
| A | US 4982611 A (WISCONSIN ALUMNI RESEARCH FOUNDATION) 08 January 1991 (1991-01-08) <br> entire document | 1-23 |
| A | CN 111505764 A (TSINGHUA UNIVERSITY) 07 August 2020 (2020-08-07) <br> entire document | 1-23 |
| A | CN 109029805 A (SHANGHAI JIAO TONG UNIVERSITY) 18 December 2018 (2018-12-18) <br> entire document | 1-23 |
| A | US 2019064012 A1 (TECHNISCHE UNIVERSITEIT EINDHOVEN) 28 February 2019 (2019-02-28) <br> entire document | 1-23 |

☑ Further documents are listed in the continuation of Box C. ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 May 2022** | **19 May 2022** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/113908** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 104034459 A (DALIAN UNIVERSITY OF TECHNOLOGY) 10 September 2014 (2014-09-10)<br>    entire document | 1-23 |
| A | CN 110779639 A (CHONGQING JIAOTONG UNIVERSITY) 11 February 2020 (2020-02-11)<br>    entire document | 1-23 |
| A | CN 104244808 A (UNIVERSITE LIBRE DE BRUXELLES et al.) 24 December 2014 (2014-12-24)<br>    entire document | 1-23 |

Form PCT/ISA/210 (second sheet) (January 2015)

**INTERNATIONAL SEARCH REPORT**
**Information on patent family members**

| International application No. |
| --- |
| **PCT/CN2021/113908** |

| Patent document cited in search report | | | Publication date (day/month/year) | Patent family member(s) | | | Publication date (day/month/year) |
| --- | --- | --- | --- | --- | --- | --- | --- |
| US | 4982611 | A | 08 January 1991 | None | | | |
| CN | 111505764 | A | 07 August 2020 | None | | | |
| CN | 109029805 | A | 18 December 2018 | WO | 2020006799 | A1 | 09 January 2020 |
| US | 2019064012 | A1 | 28 February 2019 | EP | 3420331 | A1 | 02 January 2019 |
| | | | | WO | 2017144675 | A1 | 31 August 2017 |
| | | | | JP | 2019512773 | A | 16 May 2019 |
| CN | 104034459 | A | 10 September 2014 | None | | | |
| CN | 110779639 | A | 11 February 2020 | None | | | |
| CN | 104244808 | A | 24 December 2014 | EP | 2833781 | A1 | 11 February 2015 |
| | | | | WO | 2013150019 | A1 | 10 October 2013 |
| | | | | US | 2016022373 | A1 | 28 January 2016 |

Form PCT/ISA/210 (patent family annex) (January 2015)